# EUROPÄISCHE PATENTSCHRIFT

(11) **EP 2 403 947 B1**
(45) Veröffentlichungstag und Bekanntmachung des Hinweises auf die Patenterteilung: **14.02.2018**
(21) Anmeldenummer: 09808976.6
(22) Anmeldetag: 13.11.2009
(51) Int. Cl.: A61K 39/12, A61K 39/00, C07K 14/005, G01N 33/569, C12N 15/81

(54) **VERFAHREN ZUR ORALEN/MUKOSALEN VAKZINIERUNG MITTELS REKOMBINANTER HEFEN**
METHOD FOR THE ORAL/MUCOSAL VACCINATION BY MEANS OF RECOMBINANT YEASTS
PROCÉDÉ DE VACCINATION PAR VOIE ORALE/MUQUEUSE À L'AIDE DE LEVURES DE RECOMBINAISON

(30) Priorität: 14.11.2008 DE 102008057451
(43) Veröffentlichungstag der Anmeldung: 11.01.2012
(73) Patentinhaber: Martin-Luther-Universität Halle-Wittenberg, 06108 Halle/Saale (DE)
(72) Erfinder: BREUNIG, Karin, 14109 Berlin (DE); BEHRENS, Sven-Erik, 06120 Halle/Saale (DE)
(74) Vertreter: Wissenbach, Gisela
(86) Internationale Anmeldenummer: PCT/DE2009/001623
(87) Internationale Veröffentlichungsnummer: WO 2010/054649

(56) Entgegenhaltungen:
- EP-A1- 1 790 332
- WO-A1-99/15900
- SCHREUDER M P ET AL: "Yeast expressing hepatitis B virus surface antigen determinants on its surface: implications for a possible oral vaccine" VACCINE, ELSEVIER LTD, GB LNKD- DOI:10.1016/0264-410X(95)00206-G, Bd. 14, Nr. 5, 1. April 1996 (1996-04-01), Seiten 383-388, XP004057292 ISSN: 0264-410X
- HUANG ET AL: "Secreted expression of the classical swine fever virus glycoprotein E<rns> in yeast and application to a sandwich blocking ELISA" JOURNAL OF VIROLOGICAL METHODS, ELSEVIER BV, NL LNKD- DOI:10.1016/J.JVIROMET.2005.08.020, Bd. 132, Nr. 1-2, 1. März 2006 (2006-03-01), Seiten 40-47, XP025030065 ISSN: 0166-0934
- NOBIRON ISABELLE ET AL: "DNA vaccination against bovine viral diarrhoea virus induces humoral and cellular responses in cattle with evidence for protection against viral challenge." VACCINE, Bd. 21, Nr. 17-18, 16. Mai 2003 (2003-05-16), Seiten 2082-2092, XP004421125 ISSN: 0264-410X
- MUSTILLI A C ET AL: "Comparison of secretion of a hepatitis C virus glycoprotein in Saccharomyces cerevisiae and Kluyveromyces lactis" RESEARCH IN MICROBIOLOGY, ELSEVIER, AMSTERDAM, NL, Bd. 150, Nr. 3, 1. April 1999 (1999-04-01) , Seiten 179-187, XP027033495 ISSN: 0923-2508 [gefunden am 1999-04-01]
- ZENKE F T ET AL: "Gal80 proteins of Kluyveromyces lactis and Saccharomyces cerevisiae are highly conserved but contribute differently to glucose repression of the galactose regulon." MOLECULAR AND CELLULAR BIOLOGY DEC 1993 LNKD- PUBMED:8246973, Bd. 13, Nr. 12, Dezember 1993 (1993-12), Seiten 7566-7576, XP007913836 ISSN: 0270-7306 in der Anmeldung erwähnt

## Beschreibung

Die Eindämmung von Infektionskrankheiten ist noch eine der großen Herausforderungen sowohl in der Human- als auch in der Veterinärmedizin. Besonders komplex stellt sich die Situation bei viralen Infektionen dar, die, im Gegensatz zu bakteriellen Infektionen in der Regel nicht mit Breitband-Wirkstoffen bekämpft werden können und große wirtschaftliche Schäden verursachen. Der Entwicklung neuer effektiver Impfstrategien gegen virale Erkrankungen kommt deshalb eine herausragende Bedeutung zu.

Traditionell werden zur prophylaktischen und auch zur therapeutischen Impfung gegen Viruserkrankungen "attenuierte", d.h. durch Mutagenese veränderte Viren mit deutlich verminderter bzw. abwesender Virulenz ("Lebendvakzine"), oder inaktivierte Viren ("Totvakzine"), eingesetzt. In jüngerer Zeit wurden zunehmend auch sog. "*subunit* Vakzine" bzw. "*subunit* Markervakzine" etabliert, wobei definierte gentechnisch hergestellte "Hauptantigene" des Pathogens zur Impfung eingesetzt werden. Der Begriff "Markervakzin" impliziert, dass sich vakzinierte Individuen durch nachfolgende diagnostische Analyse eindeutig von natürlich infizierten Individuen unterscheiden lassen ("DIVA"-*differentiate infected and vaccinated animals*). Unter Hauptantigenen versteht man zum Beispiel Proteine der Virushülle oder des Virus-Kapsids, die in Abwesenheit eines kompletten Viruspartikels eine humorale und/oder zelluläre Immunantwort im Wirt induzieren können, in Folge derer eine Virusinfektion präventiv oder therapeutisch abgewehrt bzw. bekämpft werden kann. Eine "*subunit* Vakzinierung" setzt voraus, dass Hauptantigene charakterisiert sind. Der Herstellung (Expression) und immunogenen Formulierung solcher Proteine ("Antigenformulierung") für den Impfprozess kommt eine Schlüsselrolle zu, insbesondere da Virus-Hüllproteine meist nicht wasserlöslich sind und nur selten in bakteriellen Expressionssystemen rekombinant hergestellt werden können. Entsprechend aufwändig sind Verfahren, "subunit" Vakzine in gereinigter Form zu gewinnen und deren Haltbarkeit zu sichern.

Hefen als Expressionssysteme für rekombinante Proteine vereinen die ökonomischen Vorteile der bakteriellen Systeme mit der für höhere Zellen typischen Organisationsform. Sie sind durch intrazelluläre Membransysteme kompartimentiert, was sie grundlegend von bakteriellen Wirtssystemen unterscheidet und die Expression membranverankerter virale Hüllproteine oder sogar ganze Viruskapside ermöglicht. Zudem haben die beta-Glucane, Glucose-Polymere aus denen die Hefe-Zellwand aufgebaut ist, eine seit langem bekannte immun-stimulierende Wirkung. Es stand daher die Aufgabe, ein Verfahren zu entwickeln, bei dem komplette Hefen zur Vakzinierung eingesetzt werden können. Das Problem wurde gelöst, indem mittels gentechnischer Methoden Gene für immunogene Determinanten in das Genom von nicht-pathogenen Hefen eingeschleust und exprimiert werden und diese rekombinanten Hefezellen direkt zur oralen/mukosalen Vakzinierung eingesetzt werden.

### Kluyveromyces lactis.

*Kluyveromyces lactis* gehört zu den sog. ***food grade*** Hefen mit dem ***GRAS* Status** (***g**enerally **r**egarded* **a**s ***s**afe*). Wie die Bäckerhefe, die als Nahrungsmittelzusatz über Jahrtausende erprobt und bewährt ist, gilt auch die in Molkereiprodukten häufig vertretene Hefe *K. lactis* für die Lebensmittelindustrie als unbedenklich.

### Orale/mukosale Impfung

Impfstoffe/Antigene können **parenteral** oder **oraler/mukosal** appliziert werden. Während im ersten Fall die Antigene unter Umgehung des Verdauungstraktes, meist durch Injektion, in die Blutbahn gelangen, verläuft bei der oralen/mukosalen Impfung die Aufnahme über das mukosale Darmepithel nach Passieren des Magens. Die Darmmukosae sind permanent pathogen-exponiert und stellen *per se* eine erhebliche Aufnahmebarriere für Infektiva dar. Darüber hinaus existiert ein mukosales Immunsystem, das in die sog. *inductive* und *effector sites,* unterschieden wird. Die Aufnahme von Antigen erfolgt in den *inductive sites* über M-Zellen in den "Peyer's patches". An die Peyer's patches assoziierte Immunzellen des MALT (*mucosa-associated lymphoid tissue*) induzieren die Immunantwort. Nach Abklingen der Immunantwort entstehen im Gesamtorganismus, als auch in den *effector sites* der Mucosae spezifische *memory* Immunzellen, die i.d.R. langwährenden Schutz gegen das ursprüngliche Antigen/Pathogen vermitteln.

**Vorteile oraler/mukosaler Impfverfahren.** Verglichen mit einer parenteralen Immunisierung erfordert die orale/mukosalen Immunisierung den Einsatz erheblich höherer Quantitäten an Antigen. Durch orale/mukosale Vakzinierung ist es im Gegensatz zur parenteralen Impfung jedoch möglich, zusätzlich zur systemischen Immunantwort auch eine lokale Immunantwort an den *effector sites* der Mukosae zu induzieren. Speziell bei Pathogenen (wie z.B. das Virus der bovinen viralen Diarrhoe, BVDV und das Virus der Klassischen Schweinepest, CSFV; siehe auch unten), die (auch) über Schleimhäute übertragen werden, hat eine orale/mukosale Vakzinierung das Potenzial, eine aktive und lang anhaltende Immunität zu erzeugen. Weitere wichtige Vorteile der oralen Vakzinierung sind eine gute Akzeptanz und Ökonomie. Im günstigsten Fall können die Vakzine mit geringen Kosten hergestellt und unkompliziert mit der Nahrung verabreicht werden.

Die orale Verabreichung von Hefe-Stämmen, die Virus-Antigene exprimieren, ist daher nicht nur unbedenklich, sondern könnte zusätzliche gesundheitsförderliche und adjuvante Effekte haben

Es stand nunmehr die Aufgabe ein Expressionssystem zu entwickeln, das die gezielte Integration von Fremdgenen in das Genom von ***Kluyveromyces lactis*** erlaubt und entsprechend die intrazelluläre Synthese von Antigenen ermöglicht. Weitere Aufgabe war es, einen solchen rekombinanten *K.lactis* Stamm, der ein spezifisches Virusantigen enthält, für eine oral/mukosale Impfung einzusetzen.

Erfindungsgemäß wurde ein Wirts-/Vektorsystem entwickelt, das es erlaubt über gentechnische Verfahren Fremdgene gezielt am *LAC4* Locus des *K*. *lactis* Genoms zu integrieren, ohne dass zusätzliche DNA-Sequenzen (Selektionsmarker o.ä.) eingeführt werden müssen. Die Fremdgenexpression steht unter Kontrolle des *LAC4* Promotors und kann durch Lactose oder Galactose im Wachstumsmedium dosiert induziert oder, nach Ausschalten des Regulatorgens, *KIGAL80* konstitutiv aktiviert werden. Die rekombinanten Hefestämme sind ohne Selektionsdruck stabil und können unter Fermentationsbedingungen zu hohen Dichten kultiviert werden. Die Fremdproteinexpression kann indirekt über die Expression eines endogenen Reportergens quantifiziert werden.

Es wurde eine Serie rekombinanter *K*. *lactis* Vakzinstämme generiert. Generell exprimieren diese Stämme induzierbar signifikante Mengen eines Proteins, oder Domänen dieses Proteins, oder Domänen dieses Proteins fusioniert mit artfremden Proteindomänen. Die dabei verwandten artfremden Proteindomänen dienen Adjuvanzzwecken bzw. der gezielten Kompartimentierung des exprimierten Fremdproteins in der Hefezelle. Neben Adjuvanzeffekten ist die Kompartimentierung des exprimierten Fremdproteins wichtig für die Optimierung der Expression bzw. die Formulierung des Expressionsproduktes. Ein rekombinanter *K.lactis* Stamm (siehe Ausführungsbeispiele) wurde erfolgreich zur oral/mukosalen Impfung eingesetzt.

Die Aufgabe der Erfindung wurde gelöst durch Bereitstellung eines rekombinanten Stammes der Hefe *Kluyveromyces lactis* gemäß Anspruch 20 und eines Verfahrens zur oralen/mukosalen Vakzinierung gemäß Anspruch 1, wobei dieses Verfahren den erfindungsgemäßen rekombinanten Stamm der Hefe *Kluyveromyces lactis* umfasst. Die Erfindung betrifft außerdem die Verwendung des rekombinanten Stammes der Hefe *Kluyveromyces lactis* zur Fremdproteinexpression sowie in der vakzinierung, insbesondere gegen das BVD Virus oder das CSF Virus, gemäß den Ansprüchen 21 bis 23.

### Ausführungsbeispiele:

### 1. Erzeugung des K. lactis Stammes VAK367-D4 (Met⁻ ura3 Iac4::ScURA3).

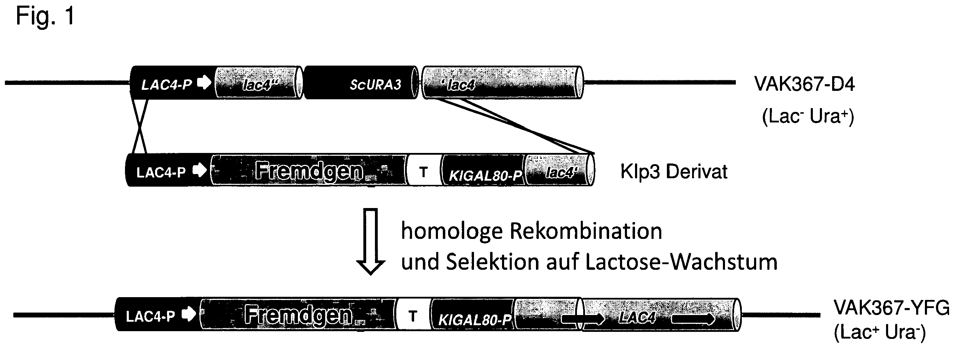

Der Ausgangsstamm VAK367 zur heterologen Expression von Fremdproteinen hat folgende Eigenschaften: Er erlaubt die Kultivierung zu hoher Zelldichte, ohne dass dabei intrazelluläre Proteine nachweisbar freigesetzt werden. Diesbezüglich unterscheidet sich dieser Stamm von vielen nahe verwandten *K*. *lactis* Stämmen. Der Stamm VAK367 wurde durch zwei Mutageneserunden von dem Stamm CBS 2359 (Centraalbureau voor Schimmelcultures http://www.fungalbiodiversitycentre.com) abgeleitet und ist auxotroph für die Aminosäure Methionin und die Nucleobase Uracil. Vom Stamm VAK367 wurde mit gentechnischen Methoden der Stamm VAK367-D4 (hinterlegt bei der Deutschen Sammlung von Mikroorganismen und Zellkulturen GmbH (DSMZ) in Braunschweig) abgeleitet, in dem mit Hilfe des Plasmids pD4-2 die Sequenz von +358 bis +1181 des *LAC4* Gens durch das *ScURA3* Gen ersetzt wurde. Der Stamm VAK367-D4 erlaubt nun die Integration von Fremdgenen am *LAC4* Locus ohne zusätzliche Marker, indem auf Lactose-Wachstum selektiert wird. Dabei wird bei Benutzung eines geeigneten Integrationsvektors, wie z.B. Klp3 (s.u.) durch homologe Rekombination die Disruptionskassette so ersetzt, dass unter Verlust des *ScURA3* Markers ein intaktes *LAC4* Gen rekonstituiert wird. (Abb.1).

### 2. Erzeugung eines Integrationsvektors, der die induzierbare Expression von Fremdgenen erlaubt.

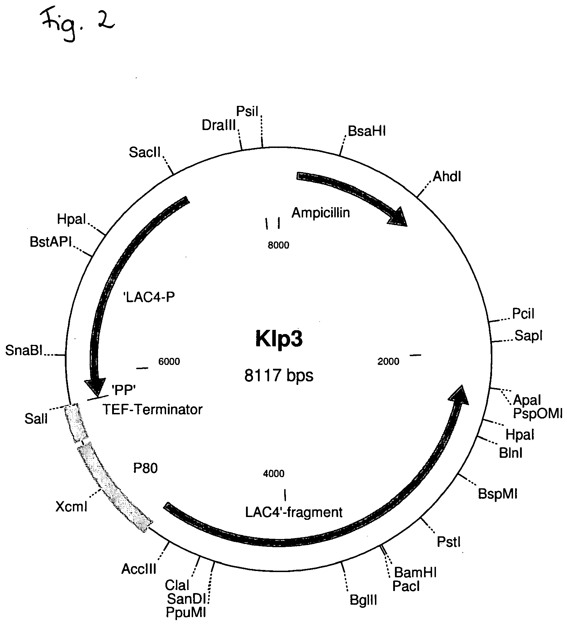

### Vektor: Klp3 (Abb. 2)

Bei dem Vektor Klp3 handelt es sich um einen *E.coli* Vektor, basierend auf YRp7, der in Hefen nicht autonom replizieren kann, da die ARS1 Sequenz deletiert wurde. Klp3 enthält *K.lactis* Sequenzen, die die Integration am *LAC4* Locus durch homologe Rekombination ermöglichen (upstream Region von *LAC4* und 5' Ende des *LAC4* Leserahmens). Zwischen *LAC4* Promotor und Transkriptionsstart wurde ein DNA Abschnitt inseriert, der den *TEF1* Terminator und den *KIGAL80* Promotor enthält. Dadurch steht der *LAC4* Leserahmen unter Kontrolle des *KIGAL80* Promotors, der über den Transkriptionsfaktor KlGal4 mit dem *LAC4* Promotor ko-reguliert wird (Zenke et al. 1993, Molecular and Cellular Biology, 13:7566-7576). Diese Konstruktion ermöglicht es, über Messung der *LAC4* codierten β-Galactosidase, die Induktion der Fremdgenexpression zu verfolgen. Klp3 erlaubt die Integration des Fremdgens zwischen *LAC4* Promotor und *TEF1* Terminator über die unikale Sa/I Schnittstelle. Zur Integration der Expressionskassette wird Klp3 mit *Hpa*I oder *EcoR*I verdaut und in *K*. *lactis* VAK367-D4 transformiert. Dabei wird die Expressionskassette von dem *E.coli* Vektoranteil getrennt, so dass die resultierenden Stämme keine bakteriellen Sequenzen enthalten..

### Plasmid KIp3-E2-1 (9437 bp)

Das BVDV Gensegment, das u.a. den für das BVDV E2 kodierenden Bereich (BVDV E2-ORF) enthielt, wurde als *Sal*I*-Xho*I Fragment in die *Sal*I Schnittstelle zwischen *LAC4* Promotor und *TEF1*-Terminator inseriert. Stromabwärts liegt der *KIGAL80* Promotor, der mit dem 5'-Ende des *LAC4* ORFs fusioniert wurde. Durch Restriktion des Plasmids mit *Hpa*I erhält man zwei Fragmente, von denen das größere die Expressionskassette mit dem BVDV E2-ORF trägt.
**3. Formulierung der E2 Hauptantigene von BVDV und CSFV.** Von BVDV (bovine viral diarrhea virus), dem Erreger der bovinen viralen Diarrhoe und der mucosal disease (BVD/MD) als auch von CSFV (classical swine fever virus), dem Erreger der klassischen Schweinepest (CSF), ist ein Hauptantigen charakterisiert. Dabei handelt es sich um das "envelope" (Virushülle-integrierte) Protein E2. E2 induziert auch in Abwesenheit eines Viruspartikels eine massive humorale Immunantwort, d.h. die Bildung effektiv virus-neutralisierender Antikörper. Durch gentechnische Formulierung von E2 konnte das immunogene Potential des Proteins weiter verstärkt und auch eine zelluläre Immunantwort erzeugt werden (Bruschke et al., 1999; Nobiron et al., 2003). Die spezifische und exklusive Immunantwort gegen einzelne, unter Umständen, gentechnisch formulierte Proteindomänen des E2 erlaubt die Diskriminierung vakzinierter Tiere von mit Feldvirus infizierten Tieren, z.B. mittels ELISA-Verfahren.

### 4. Konstruktion eines K. lactis Stammes, der das BVDV E2 Protein exprimiert

Mittels der erfindungsgemäßen Technologie wurde *K. lactis* Stamm, VAK367-E2-1, hergestellt. Dazu wurde das Plasmid Klp3-E2-1 mit *Hpa*I geschnitten und das größere *Hpa*I Fragment, das den BVDV E2-ORF kodiert, über homologe Rekombination chromosomal in den Ausgangsstamm VAK367-D4 (DSM 23097) integriert. In VAK367-D4 (DSM 23097) ist das *LAC4* Gen durch Insertion des *URA3* Gens zerstört (*lac4::URA3*)*.* Durch die chromosomale Integration des genannten Fragmentes wurde der Genort *lac4::URA3* ersetzt und das intakte *LAC4* Gen rekonstituiert (Abb. 1). Die Selektion von *K*. *lactis* Zellen, die die BVDV Gensequenz enthielten, erfolgte durch Wachstum auf Lactose Medium. Der Verlust des *URA3* Gens wurde durch den Ura-minus Phänotyp bestätigt. Die Sequenz des entsprechenden Genortes wurde durch DNA-Sequenzierung bestätigt. (Sequenzprotokoll 1).

Bei dem resultierenden Stamm VAK367-E2-1 ist ein Segment des BVDV Genoms aus BVDV Stamm CP7 in das Hefegenom integriert. Das entsprechende BVDV Gensegments enthielt den Bereich, der für das E2 Protein sowie Teile der angrenzenden E1- und p7- Regionen kodiert. Die E1- und p7-Regionen enthalten die für die korrekte Prozessierung des E2-Proteins (Sequenzprotokoll 2) notwendigen Signalsequenzen. Die korrekte Prozessierung (Maturierung) des BVDV E2 Proteins erfolgt über Signalasen, die von der Hefezelle kodiert werden.

Mittels eines spezifisch entwickelten Immunofluoreszenz-Detektionsverfahrens kann die Expression von BVDV E2 in Zellen des K. *lactis* Stammes VAK367-E2-1 festgestellt werden. Ein speziell für den BVDV E2 Nachweis entwickeltes ELISA-Verfahren erlaubt die Detektion und Quantifizierung von heterolog exprimiertem Antigen. Das analoge ELISA-Verfahren konnte zur exakten Quantifizierung des Antikörpertiters immunisierter Tiere eingesetzt werden. Virusneutralisationsverfahren und Verfahren zur Charakterisierung von Antikörpern und T-Zellen wurden als Routineverfahren eingesetzt.

Ein neuartiges qRT-PCR Verfahren ermöglicht es, das BVDV RNA Genom aus Serum und Zellkulturüberständen nachzuweisen und zu quantifizieren.

### 5. Nachweis der Wirksamkeit von K.lactis Stamm VAK367-E2-1 in oralen/mukosalen Immunisierungstudien

### Studie 1.

In Tierversuchen wurde einer signifikanten Zahl von Versuchsmäusen eine Emulsion naiver *K*. *lactis* unter standardisierten Bedingungen appliziert. Dabei wurden unterschiedliche Immunisierungsschemata verwendet. Hauptkriterien waren dabei unterschiedliche Quantitäten zugeführter Hefe (3 - max. 8% Anteil der täglich eingenommenen Nahrung) und unterschiedliche "Boosterintervalle".

### Ergebnisse:

(i) Es wurde eine generelle Verträglichkeit von *K.lactis* demonstriert: die orale Gabe der Hefeemulsionen bewirkte keine sichtbaren Veränderungen im Befinden der Tiere.
(ii) Die orale Gabe von *K.lactis* führte zu einer eindeutig detektierbaren humoralen Immunantwort gegen bestimmte Hefeproteine. Durch Westernblot-Verfahren konnte bei den mit *K.lactis* gefütterten Tieren im Vergleich zu Kontrolltieren eine signifikante und spezifische Antikörperantwort gegen Hefeproteine festgestellt werden. Hefeproteine haben also *per se* eine immunogene Wirkung. Neben dem *proof of principle,* dass mit der oralen Applikation von *K.lactis* eine Immunantwort erzeugt werden kann, wurde indiziert, dass durch die orale Gabe von Hefe in Kombination mit einem rekombinanten Antigen ein zusätzlicher Adjuvanzeffekt erzielt werden kann.

### Studie 2

In weiteren Tierversuchen mit einer signifikanten Anzahl an Mäusen wurde mit einem optimierten Immunisierungsschema (siehe Studie 1) und mit rekombinanter *K*. *lactis* des Stammes 367-E2-1 oral vakziniert.

### Ergebnisse:

(i) Über das spezielle ELISA-Verfahren konnte die Bildung von anti-BVDV E2 Antikörpern in den mit *K.lactis* Stamm 367-E2-1 vakzinierten Mäusen im Vergleich zu Kontrollmäusen (immunisiert mit naiver *K.lactis*) detektiert werden
ii) In einem Neutralisationstest mit BVDV auf bovinen Kulturzellen konnte eine neutralisierende Wirkung von Antiseren aus Mäusen, die mit *K*. *lactis* des Stammes 367-E2-1 immunisiert wurden, wiederum im Vergleich zu Seren aus Kontrollmäusen festgestellt werden.
iii) Durch die Verwendung von Adjuvanzien wie CpG-ODN und QuilA wurde eine Verstärkung der Immunantwort erreicht.
iv) Durch mukosale/orale Immunisierung mit dem rekombinanten *K.lactis* Stamm VAK367-E2-1 konnte eine wirksame Protektion erreicht werden. Dies konnte in Challenge-Experimenten mit rekombinanten Vakziniaviren, die das BVDV E2-Protein exprimieren, gezeigt werden.

### Die Ergebnisse der Studien belegen, dass erfindungsgemäß mittels

Erzeugung eines *K.lactis* Stammes und Varianten dieses Stammes, Fremdgene ohne weitere Selektionsmarker gezielt in diese integriert werden können. Die resultierenden rekombinanten Abkömmlinge des betreffenden *K lactis* Stammes können zu Expressionszwecken für die nachfolgende biochemische Darstellung eines heterologen Proteins verwendet werden. Die Zellen der entsprechenden rekombinanten *K.lactis* Stämme können direkt zur mukosalen/oralen oder parenteralen Immunisierung eingesetzt werden.

### Sequence Data

Molecule: KIp3, 8117 bps DNA Circular
Description: Ligation of lac4 into i-amp
File Name: KIp3.cm5, dated 12 Nov 2009
Printed: 1-8117 bps (Full), format Single Strand

1 aggtggcact tttcggggaa atgtgcgcgg aacccctatt tgtttatttt tctaaataca
61 ttcaaatatg tatccgctca tgagacaata accctgataa atgcttcaat aatattgaaa
121 aaggaagagt atgagtattc aacatttccg tgtcgccctt attccctttt ttgcggcatt
181 ttgccttcct gtttttgctc acccagaaac gctggtgaaa gtaaaagatg ctgaagatca
241 gttgggtgca cgagtgggtt acatcgaact ggatctcaac agcggtaaga tccttgagag
301 ttttcgcccc gaagaacgtt ttccaatgat gagcactttt aaagttctgc tatgtggcgc
361 ggtattatcc cgtattgacg ccgggcaaga gcaactcggt cgccgcatac actattctca
421 gaatgacttg gttgagtact caccagtcac agaaaagcat cttacggatg gcatgacagt
481 aagagaatta tgcagtgctg ccataaccat gagtgataac actgcggcca acttacttct
541 gacaacgatc ggaggaccga aggagctaac cgcttttttg cacaacatgg gggatcatgt
601 aactcgcctt gatcgttggg aaccggagct gaatgaagcc ataccaaacg acgagcgtga
661 caccacgatg cctgtagcaa tggcaacaac gttgcgcaaa ctattaactg gcgaactact
721 tactctagct tcccggcaac aattaataga ctggatggag gcggataaag ttgcaggacc
781 acttctgcgc tcggcccttc cggctggctg gtttattgct gataaatctg gagccggtga
841 gcgtgggtct cgcggtatca ttgcagcact ggggccagat ggtaagccct cccgtatcgt
901 agttatctac acgacgggga gtcaggcaac tatggatgaa cgaaatagac agatcgctga
961 gataggtgcc tcactgatta agcattggta actgtcagac caagtttact catatatact
1021 ttagattgat ttaaaacttc atttttaatt taaaaggatc taggtgaaga tcctttttga
1081 taatctcatg accaaaatcc cttaacgtga gttttcgttc cactgagcgt cagaccccgt
1141 agaaaagatc aaaggatctt cttgagatcc tttttttctg cgcgtaatct gctgcttgca
1201 aacaaaaaaa ccaccgctac cagcggtggt ttgtttgccg gatcaagagc taccaactct
1261 ttttccgaag gtaactggct tcagcagagc gcagatacca aatactgtac ttctagtgta
1321 gccgtagtta ggccaccact tcaagaactc tgtagcaccg cctacatacc tcgctctgct
1381 aatcctgtta ccagtggctg ctgccagtgg cgataagtcg tgtcttaccg ggttggactc
1441 aagacgatag ttaccggata aggcgcagcg gtcgggctga acggggggtt cgtgcacaca
1501 gcccagcttg gagcgaacga cctacaccga actgagatac ctacagcgtg agctatgaga
1561 aagcgccacg cttcccgaag ggagaaaggc ggacaggtat ccggtaagcg gcagggtcgg
1621 aacaggagag cgcacgaggg agcttccagg gggaaacgcc tggtatcttt atagtcctgt
1681 cgggtttcgc cacctctgac ttgagcgtcg atttttgtga tgctcgtcag gggggcggag
1741 cctatggaaa aacgccagca acgcggcctt tttacggttc ctggcctttt gctggccttt
1801 tgctcacatg ttctttcctg cgttatcccc tgattctgtg gataaccgta ttaccgcctt
1861 tgagtgagct gataccgctc gccgcagccg aacgaccgag cgcagcgagt cagtgagcga
1921 ggaagcggaa gagcgcccaa tacgcaaacc gcctctcccc gcgcgttggc cgattcatta
1981 atgcagctgg cacgacaggt ttcccgactg gaaagcgggc agtgagcgca acgcaattaa
2041 tgtgagttag ctcactcatt aggcacccca ggctttacac tttatgctcc cggctcgtat
2101 gttgtgtgga attgtgagcg gataacaatt tcacacagga aacagctatg accatgatta
2161 cgccaagcgc gcaattaacc ctcactaaag ggaacaaaag ctgggtaccg ggcccgcgac
2221 ctaaccattc aaatgattca taactatctc ctagccagaa ttcgtaccca actcttggga
2281 aatcaggagg ctgatattcg ccagtaagct tcatagaagt gttcaagttt attttgttag
2341 caaagatcgt gtacttctga acagtctcaa acccatagta aaatacaact ggggatatac
2401 gagagttaac cgtgactaca gctagagaac cattagaacc tttttcgaca ctcactccat
2461 ggatgttttg cttcattaaa tcaatattgt acttcttcca gttcttaaag tccctaggtt
2521 catcattatt cgttggaggt ctccagaaag tgattgaaga accctcaaac ttgctggaaa
2581 tttccttacc cttgaccttt aggctttcaa ttttacccaa caatttgtcc aagataaaat
2641 gcaatccact ggattcaact gagacataac gtttaccgtc gttgatcttc gcagcttttt
2701 ctgctgtctc tgtaacaaaa tcgggtacct tcaatggaag ttcagcttgg ccccaggcaa
2761 tttcatgacc tgcctttaga acaccagcat catctttcaa cacggcaaca acataagttg
2821 tatcagaagg aatagtaaca gattcttctg gctttaaaga tggaacgtcg attgtctttc
2881 ccgtgtcctt gtcgataaac aataagtggt ctgtcgtaat gaagtcgtgc ttatttgtga
2941 ttgttacaga tccgtgcgca attttaatat gaacgggttc aataaccttc ttatactcta
3001 caaggcccgg agtaggatta tgctcactgt tacacaaacc atccatgatg aacactccgt
3061 catgaacctc ttccttaaag tcaccaccat aagcataagc tttatgcaac ttaccatctg
3121 cagtactaac atcttcgaat tcaataccgt gatttgccca ttcccagata aagccacctt
3181 ggtaaaactt ctccttgtag aacaactctt gatattcttt caaagagcca ggaccgttac
3241 ccattgcatg gccgtactca cacaagatca aaggcttttc aaacttacca ttttcatcag
3301 tgtggttctt cctccacctt tccataattt caaatgttgg gtacatgaaa ctaaagatat
3361 ctgcactcaa agcgttcaag tcaccctcat aatgcacaag tctggtagga tccaattgtt
3421 taattaactt gtacatggct ttgtggtttc tgccataaca agcttcgtta cccaaggacc
3481 agataataat cgaaggatga ttgacatctc ttaggacaag ttgggaagct ctgtctaagt
3541 acgcgacctc gtactctgga ttatctgata agtaatgggc attaacatcg tagagtttat
3601 ttttagtatc tggatattca gcctccaagt tcgtatgacg attaaatggc tcttgaacac
3661 catgagtttc aagatctgcc tcgtcaatga cccagaagcc cagcttatcg aagaggtcat
3721 acaccttagg atggtttgga taatgcgagt tacgaacagc attgatgtta aacttcttca
3781 ttagaatcaa gtccctaaca acaaaatcta atggcacagc tctaccgaac cttggatggt
3841 gatcatgtct gttgacacct ctaaagagaa tgtctttgcc attaacagta atgttaccgt
3901 ccttcaactc cacttgtctg aaaccaacat ggtgcttaat agattgaatc acactgccat
3961 cagatccaat taaatccaac tggtacttgt acaaagtagg attttctgcg gtccaatgtt
4021 ctggggcctt gacgttgatc ttgaaagctg tttcttcgtt ctttttggtg gagaaggaaa

4081 taaattcttt agttgaaaaa gtcgtgttcc cattctcctc gttcaacaaa gagcttgcat
4141 cgtaaacttt agatccatct tcaggttcgt aaagtgtgaa attgatgtga tcataagaag
4201 aaccctggac atcaactttc acagaaagct ctgcatcctg atactgagag tccacaaaag
4261 ttgtagtgac cctaacgtct tcaatatggg ccttcttagg caattttagt aaagaaacgt
4321 ctctgtaaat accagagagc caccattgat cttggtcctc gatataagtg gaatcggacc
4381 acttgaaaac cttgacgacc actaagtttt cgccctcaga aacgtacttt tggatatcaa
4441 attcagcccc gttacgggac cccttattga aacccacata ttgaccatta acataaagct
4501 cgtaacaatt gtccacaccc tcaaatctca atctgtgctc gaacgactca atcgatttcg
4561 aatctaattc aaaagttcta gcataaacac cagtaggatt tacagtggga ggatttggga
4621 tgtcgattgg gatagggtac tgtacgttcg tgtaaattgg tttaccgtac ttccagtctt
4681 cctgaagttc ccaatgggat ggcacagaaa tggtgctcca tttctttgcc gtttcccagt
4741 ctaaattctt agcatccgga gcgtcaagag gtgcatcaaa caacgcaaaa gcccaaggcc
4801 cattgagaga ttcgaaaata tcctgatcat agtagtaagc cctagtaggc aatctatttt
4861 cgtgaacctt tttggggttc cttaaattct caggaataag gcaagccatg gtgccgtcct
4921 gccgagatat tgtgtacact ggatcaaata ataacacttt caaagtgact aaatcacaat
4981 tgtcccaaga tatactatag ctctctgttt aacctttata ttgtcaaaaa gggacaatga
5041 atgaaagtac aaacacaaac acaaacacaa tggaagggag tgtccagggt ggtgattcct
5101 gactgtactg attcgacgga gttttatttg atttcgttga agtggttaaa gtgaataatt
5161 cttgaattga gaggaacaaa gagtggataa aataacggaa tggagaggtc cgagcgatga
5221 ataatgtacg attcggaaga ctatgagccg gctgaacctg aggttatgga ccactaacgt
5281 cctggttgac aagagtagtc atgtaataca aacgtaaatg tgatatttaa tagaatataa
5341 gtagatatag ttaaaaagaa gaagaagaat agaaagaata agggtattag aaatttagag
5401 tcattttaaa caattgataa cttgggttaa agctcgaagt tttgttgata gtagtttttt
5461 tttttgtttt agttggtttg ttcaatagta taaggttaca gggtgcgaga caaacgttgt
5521 aacacttttc atctcccccc gctaatcacc tagtcgagag ctcgttttcg acactggatg
5581 gcggcgttag tatcgaatcg acagcagtat agcgaccagc attcacatac gattgacgca
5641 tgatattact ttctgcgcac ttaacttcgc atctgggcag atgatgtcga ggcgaaaaaa
5701 aatataaatc acgctaacat ttgattaaaa tagaacaact acaatataaa aaaactatac
5761 aaatgacaag ttcttgaaaa caagaatctt tttattgtca gtactgagtc gacggtacag
5821 cttctcgatg agtatgtgtg tttatttttt ttttattttt tttgccaaat tctgctcttt
5881 cctaaatttc aagtgttgag cttgttatcc gctcacaatt ccagcttttg tctcttcacc
5941 ttttccaact acaagcgcaa cataacaaaa gaataataat tctcctaaga aacacaagcc
6001 tcatatacct ttcgagttag ggaagaacat cttctctctg ttctcacatt gattcgagct
6061 attaaatacc tttttctcaa tcgaaatctc aagtaaaaca gcaatgaaaa cattacgtaa
6121 ctaaaggtgt tcaccactag aaatcatacc cttcacactc gacttcaagt agtgaatggt
6181 gtagcaacaa agtccaaata ccaatgtcaa ccaagtaacc gaccgcacta ctagaaaaag
6241 acgctgttgc tcggaccaca aatttccgct acacttttca caactatact gaagatacaa
6301 aaaacgtgtg tgggtatggc tggctaccag gtcgcctggt taaaccaagt caacgtgata
6361 catatgtacg ttccaacact aagcctaccc taagtttcgg ctcacaggct aggctattat
6421 taacatgcaa gacaagggag aagcaaagca aagaccaacc gaaacccacc agagcaccct
6481 gaactttgcg gtgaacagaa ttccgcaaca tatctgagga taccatgatc tcgttttcct
6541 actccatatg ggaatcaccc actgttgtcc gtaaatatga ccaaattcct accttgattc
6601 ctcacgaata atcgcagtcc gaaaagccgt tccaaaagcc agtccacagt ccatcaattg
6661 gtatgatgat tgtttttttg ttcaaactga cacactaacg gtgtggaatg cgaagagtga
6721 gcttaccctt ctcctctttg ctagcagtac ttgcctacct acctactcta ctacgctgcc
6781 atattgtcta acattcggct ttctctattt ctacctggcc tggatggctc cgtctcgccc
6841 gcctcacaca catacattcc tccccctctc gcctgcccca taataattaa acaagttaac
6901 aaaaggcgtt acctcttccg catcctctcc aatctcatac gattcccctt tcatccgact
6961 tacccaacaa gatacaggat ctcagtgaaa gatccttcct gccctccctg tctgttgtct
7021 actctacatg cgacttggaa ggccaaagga ctatcgcatg attattcgcc gggaacccgc
7081 gagttccctg ctcttttctt tcaaaccagg cagcaaacca ggtgaacaca ctctgatgta
7141 gtgcagtccc taagtccttt gaagattcgg ggagctagct acccacgcga atgtaacaaa
7201 agaacattta cttttgtggg gggtggaaaa gtcgattagg atcttgcagc acagaaactg
7261 cgcaggggtt tttttcatct tggagaagca actggctaaa ttcgacacaa acaaaaactg
7321 aaaaatggaa aataaaaaat gaaaaagcaa gctgaattcg aagaagggag aattccgcct
7381 tctgcaacca cactaatggt tggtagtcaa tagatacgca ttagaaggtt actattttat
7441 gagtcgatcc ccgcggtgga gctccaattc gccctatagt gagtcgtatt acgcgcgctc
7501 actggccgtc gttttacaac gtcgtgactg ggaaaaccct ggcgttaccc aacttaatcg
7561 ccttgcagca catccccctt tcgccagctg gcgtaatagc gaagaggccc gcaccgatcg
7621 cccttcccaa cagttgcgca gcctgaatgg cgaatggcgc gacgcgccct gtagcggcgc
7681 attaagcgcg gcgggtgtgg tggttacgcg cagcgtgacc gctacacttg ccagcgccct
7741 agcgcccgct cctttcgctt tcttcccttc ctttctcgcc acgttcgccg gctttccccg
7801 tcaagctcta aatcgggggc tccctttagg gttccgattt agtgctttac ggcacctcga
7861 ccccaaaaaa cttgattagg gtgatggttc acgtagtggg ccatcgccct gatagacggt
7921 ttttcgccct ttgacgttgg agtccacgtt ctttaatagt ggactcttgt tccaaactgg
7981 aacaacactc aaccctatct cggtctattc ttttgattta taagggattt tgccgatttc
8041 ggcctattgg ttaaaaaatg agctgattta acaaaaattt aacgcgaatt ttaacaaaat
8101 attaacgctt acaattt

## Patentansprüche

1. Verfahren zur oralen/mukosalen Vakzinierung umfassend die Verabreichung eines gentechnisch erzeugten rekombinanten Hefe-Stammes der Hefe *Kluyveromyces lactis* **gekennzeichnet dadurch, dass** dieser rekombinante Stamm eine genomisch integrierte Expressionskassette enthält, die zusammengesetzt ist aus dem Lactose induzierbaren *LAC4* Promotor, einem für ein immunogenes Protein kodierendes Fremdgen, einem Transkriptionsterminator und dem für das Enzym β-Galactosidase kodierenden *LAC4* Gen unter Kontrolle des *GAL80* Promotors von *K. lactis* (*KIGAL80-P*), wobei die gezielte Integration von Fremdgenen am *LAC4* Locus des Hefegenoms erfolgt, ohne dass zusätzliche DNA-Sequenzen eingeführt werden, und die Expression von Fremdgenen durch Lactose oder Galactose dosiert induziert oder, nach Ausschalten des Regulatorgens *KIGAL80,* konstitutiv aktiviert wird.

2. Verfahren nach Anspruch 1, **gekennzeichnet dadurch, dass** die rekombinanten *K. lactis* Stämme bei Wachstum unter nicht-selektiven Bedingungen genetisch stabil sind.

3. Verfahren nach Anspruch 1, **gekennzeichnet dadurch, dass** die rekombinanten Stämme der Hefe *Kluyveromyces lactis* als Expressionssystem und zur Vakzinierung eingesetzt werden.

4. Verfahren nach Anspruch 1 oder 2, **gekennzeichnet dadurch, dass** die rekombinanten Stämme von *Kluyveromyces lactis,* die durch Lactose induzierbare Expression von Fremdgenen erlauben.

5. Verfahren nach einem oder mehreren der oben genannten Ansprüche 1 bis 3 **gekennzeichnet dadurch, dass** das Fremdgen am *LAC4* Locus des rekombinanten *Kluyveromyces lactis* Stammes integriert wird.

6. Verfahren nach einem oder mehreren der oben genannten Ansprüche **gekennzeichnet dadurch, dass** der rekombinante *Kluyveromyces lactis* Stamm außer dem Fremdgen keine zusätzlichen Vektorsequenzen oder Selektionsmarker enthält.

7. Verfahren nach einem oder mehreren der oben genannten Ansprüche **gekennzeichnet dadurch, dass** die Fremdgenexpression nach Ausschalten des *KIGAL80* Gens konstitutiv erfolgt

8. Verfahren nach einem oder mehreren der oben genannten Ansprüche **gekennzeichnet dadurch, dass** die Fremdgenexpression indirekt über die Expression eines endogenen Reportergens quantifiziert werden kann.

9. Verfahren nach einem oder mehreren der oben genannten Ansprüche **gekennzeichnet dadurch, dass** das Fremdgen die Expression viraler Proteine mit antigenen Eigenschaften ermöglicht.

10. Verfahren nach einem oder mehreren der oben genannten Ansprüche **gekennzeichnet dadurch, dass** das Fremdgen die Expression von viralen Strukturproteinen ermöglicht.

11. Verfahren nach einem oder mehreren der oben genannten Ansprüche **gekennzeichnet dadurch, dass** das Fremdgen die Expression von viralen Strukturproteinen der Familien *Flaviviridae, Birnaviridae* und *Orthomyxoviridae* ermöglicht.

12. Verfahren nach einem oder mehreren der oben genannten Ansprüche **gekennzeichnet dadurch, dass** das Fremdgen die Expression von Strukturproteinen des Virus der bovinen viralen Diarrhoe (BVDV) ermöglicht.

13. Verfahren nach einem oder mehreren der oben genannten Ansprüche **gekennzeichnet dadurch, dass** die rekombinanten Stämme der Hefe *Kluyveromyces lactis* induzierbar oder konstitutiv signifikante Quantitäten eines Proteins oder Domänen dieses Proteins oder Domänen dieses Proteins fusioniert mit artfremden Proteindomänen exprimieren.

14. Verfahren nach einem oder mehreren der oben genannten Ansprüche **gekennzeichnet dadurch, dass** die biochemische Darstellung von Fremdproteinen ermöglicht wird.

15. Verfahren nach einem oder mehreren der oben genannten Ansprüche **gekennzeichnet dadurch, dass** bei einer Vakzinierung mittels der Administration von kompletten Hefezellen eines rekombinanten Stammes der Hefe *Kluyveromyces lactis* eine spezifische Immunisierung gegen exprimiertes Fremdprotein erzeugt wird.

16. Verfahren nach Anspruch 15 **gekennzeichnet dadurch, dass** bei einer Vakzinierung mittels inhalierender, bronchialer, oraler, nasaler Administration kompletter Hefezellen eines rekombinanten Stammes der Hefe *Kluyveromyces lactis* eine spezifische Immunisierung gegen exprimiertes Fremdprotein erzeugt wird.

17. Verfahren nach Anspruch 16, **gekennzeichnet dadurch, dass** biochemisch dargestellte Fremdproteine eine spezifische Immunisierung ermöglichen.

18. Rekombinanter Stamm der Hefe *Kluyveromyces lactis,* **gekennzeichnet dadurch, dass** dieser rekombinante Stamm eine genomisch integrierte Expressionskassette enthält, die zusammengesetzt ist aus dem Lactose induzierbaren *LAC4* Promotor, einem für ein immunogenes Protein kodierendes Fremdgen, einem Transkriptionsterminator und dem für das Enzym β-Galactosidase kodierenden *LAC4* Gen unter Kontrolle des *GAL80* Promotors von *K. lactis* (*KIGAL80-P*), wobei die gezielte Integration von Fremdgenen am *LAC4* Locus des Hefegenoms erfolgt, ohne dass zusätzliche DNA-Sequenzen eingeführt werden, und die Expression von Fremdgenen durch Lactose oder Galactose dosiert induziert oder, nach Ausschalten des Regulatorgens *KIGAL80,* konstitutiv aktiviert wird.

19. Verwendung des rekombinanten Stammes der Hefe *Kluyveromyces lactis* nach Anspruch 18 zur Fremdproteinexpression.

20. Rekombinanter Stamm der Hefe *Kluyveromyces lactis* nach Anspruch 18 zur Verwendung in der Vakzinierung.

21. Rekombinanter Stamm der Hefe *Kluyveromyces lactis* nach Anspruch 18 zur Verwendung in der oralen/mukosalen Vakzinierung gegen das BVD Virus oder das CSF Virus.

## Claims

1. Method for oral/mucosal vaccination, comprising administering a genetically engineered recombinant yeast strain of the yeast *Kluyveromyces lactis,* **characterized in that** said recombinant strain contains a genomically integrated expression cassette which is composed of the lactose-inducible *LAC4* promoter, a foreign gene coding for an immunogenic protein, a transcription terminator, and the *LAC4* gene coding for the β-galactosidase enzyme under the control of the *GAL80* promoter from *K. lactis* (*KIGAL80-P*), wherein foreign genes are specifically integrated at the *LAC4* locus of the yeast genome, without any additional DNA sequences being introduced, and expression of foreign genes is induced by metered lactose or galactose or is constitutively activated after the *KIGAL80* regulator gene has been switched off.

2. Method according to Claim 1, **characterized in that** the recombinant *K. lactis* strains are genetically stable when growing under non-selective conditions.

3. Method according to Claim 1, **characterized in that** the recombinant strains of *Kluyveromyces lactis* yeast are employed as expression system and for vaccination.

4. Method according to Claim 1 or 2, **characterized in that** the recombinant strains of *Kluyveromyces lactis* allow lactose-inducible expression of foreign genes.

5. Method according to one or more of the preceding Claims 1 to 3, **characterized in that** the foreign gene is integrated at the *LAC4* locus of the recombinant *Kluyveromyces lactis* strain.

6. Method according to one or more of the preceding claims, **characterized in that** the recombinant *Kluyveromyces lactis* strain does not contain any additional vector sequences or selection markers besides the foreign gene.

7. Method according to one or more of the preceding claims, **characterized in that** the foreign genes are constitutively expressed, after switching-off of the *KIGAL80* gene.

8. Method according to one or more of the preceding claims, **characterized in that** expression of foreign genes may be quantified indirectly via expression of an endogenous reporter gene.

9. Method according to one or more of the preceding claims, **characterized in that** the foreign gene enables viral proteins with antigenic properties to be expressed.

10. Method according to one or more of the preceding claims, **characterized in that** the foreign gene enables viral structural proteins to be expressed.

11. Method according to one or more of the preceding claims, **characterized in that** the foreign gene enables viral structural proteins of the *Flaviviridae, Birnaviridae* and *Orthomyxoviridae* families to be expressed.

12. Method according to one or more of the preceding claims, **characterized in that** the foreign gene enables structural proteins of bovine viral diarrhoea virus (BVDV) to be expressed.

13. Method according to one or more of the preceding claims, **characterized in that** the recombinant strains of *Kluyveromyces lactis* yeast inducibly or constitutively express significant quantities of a protein or domains of said protein oder domains of said protein fused to protein domains of other species.

14. Method according to one or more of the preceding claims, **characterized in that** foreign proteins can be biochemically presented.

15. Method according to one or more of the preceding claims, **characterized in that** vaccination by administration of complete yeast cells of a recombinant *Kluyveromyces lactis* yeast strain generates specific immunization against expressed foreign protein.

16. Method according to Claim 15, **characterized in that** vaccination by inhaling, bronchial, oral, nasal administration of complete yeast cells of a recombinant *Kluyveromyces lactis* yeast strain generates specific immunization against expressed foreign protein.

17. Method according to Claim 16, **characterized in that** biochemically presented foreign proteins make specific immunization possible.

18. Recombinant strain of *Kluyveromyces lactis* yeast, **characterized in that** said recombinant strain contains a genomically integrated expression cassette which is composed of the lactose-inducible *LAC4* promoter, a foreign gene coding for an immunogenic protein, a transcription terminator, and the *LAC4* gene coding for the β-galactosidase enzyme under the control of the *GAL80* promoter from *K. lactis* (*KIGAL80-P*), wherein foreign genes are specifically integrated at the *LAC4* locus of the yeast genome, without any additional DNA sequences being introduced, and expression of foreign genes is induced by metered lactose or galactose or is constitutively activated after the *KIGAL80* regulator gene has been switched off.

19. Use of the recombinant strain of *Kluyveromyces lactis* yeast according to Claim 18 for expression of foreign proteins.

20. Recombinant strain of *Kluyveromyces lactis* yeast according to Claim 18 for use in vaccination.

21. Recombinant strain of *Kluyveromyces lactis* yeast according to Claim 18 for use in oral/mucosal vaccination against BVD virus or CSF virus.

## Revendications

1. Procédé de vaccination orale/muqueuse incluant l'administration d'une souche de levure recombinante de la levure *Kluyveromyces lactis* créée par génie génétique en ce que cette souche recombinante contient une cassette d'expression intégrée au génome et est constituée du promoteur inductible du lactose LAC4, soit un gène étranger codificateur pour une protéine immunogène, un terminator de transcription et un gène LAC4 codificateur pour l'enzyme β-galactosidase sous le contrôle du promoteur GAL80 de *K*. *lactis* (KIGAL80-P). L'intégration ciblée s'effectue par les gènes étrangers sur le locus LAC4 du génome de levure sans que des séquences ADN complémentaires soient introduites ou que l'expression de gènes étrangers soient dosément induite par le lactose ou le galactose ou constitutivement activée après désactivation du gène régulateur KIGAL80.

2. Procédé selon la revendication 1, **caractérisé en ce que** les souches recombinantes *K*. *lactis* sont génétiquement stables en cas de croissance soumise à des conditions sous sélectives.

3. Procédé selon la revendication 1, **caractérisé en ce que** les souches recombinantes de levure *Kluyveromyces lactis* font office de système d'expression et sont utilisées à des fins de vaccination.

4. Procédé selon la revendication 1 ou 2, **caractérisé en ce que** les souches recombinantes de *Kluyveromyces lactis* autorisent l'expression de gènes étrangers inductibles par le lactose.

5. Procédé selon une ou plusieurs des revendications 1 à 3 citées ci-dessus, **caractérisé en ce que** le gène étranger est intégré au locus LAC4 de la souche recombinante *Kluyveromyces lactis.*

6. Procédé selon une ou plusieurs des revendications citées ci-dessus, **caractérisé en ce que** la souche recombinante *Kluyveromyces lactis* ne comprend, hormis le gène étranger, aucune séquence vectorielle ni aucun marqueur de sélection supplémentaire.

7. Procédé selon une ou plusieurs des revendications citées ci-dessus, **caractérisé en ce que** l'expression du gène étranger s'effectue de manière constitutive après désactivation du gène *KIGAL80.*

8. Procédé selon une ou plusieurs des revendications citées ci-dessus, **caractérisé en ce que** l'expression du gène étranger peut être quantifié indirectement par l'expression d'un gène rapporteur endogène.

9. Procédé selon une ou plusieurs des revendications citées ci-dessus, **caractérisé en ce que** le gène étranger permet l'expression de protéines virales au propriétés antigènes.

10. Procédé selon une ou plusieurs des revendications citées ci-dessus, **caractérisé en ce que** le gène étranger permet l'expression de protéines structurales virales.

11. Procédé selon une ou plusieurs des revendications citées ci-dessus, **caractérisé en ce que** le gène étranger permet l'expression de protéines structurales virales de la famille *Flaviviridae, Birnaviridae* et *Orthomyxoviridae.*

12. Procédé selon une ou plusieurs des revendications citées ci-dessus, **caractérisé en ce que** le gène étranger permet l'expression de protéines structurales du virus de diarrhée virale bovine (BVD).

13. Procédé selon une ou plusieurs des revendications citées ci-dessus, **caractérisé en ce que** les souches recombinantes de la levure *Kluyveromyces lactis* expriment des quantités notables d'un point de vue inductible ou constitutif d'une protéine ou de domaines de cette protéine fusionnés avec des domaines protéiques étrangers.

14. Procédé selon une ou plusieurs des revendications citées ci-dessus, **caractérisé en ce que** la représentation biochimique de protéines étrangère est possible.

15. Procédé selon une ou plusieurs des revendications citées ci-dessus, **caractérisé en ce qu'**une immunisation spécifique face à la protéine étrangère exprimée est produite en cas de vaccination effectuée par administration des cellules de levure complètes d'une souche recombinante de levure *Kluyveromyces lactis.*

16. Procédé selon la revendication 15, **caractérisé en ce qu'**une immunisation spécifique face à la protéine étrangère exprimée est produite en cas de vaccination effectuée par administration inhalatoire, bronchiale, orale ou nasale de cellules de levure complètes d'une souche recombinante de levure *Kluyveromyces lactis.*

17. Procédé selon la revendication 16, **caractérisé en ce que** les protéines étrangères représentées de façon biochimique permettent une immunisation spécifique.

18. Souche recombinante de levure *Kluyveromyces lactis,* **caractérisée en ce que** cette souche recombinante contient une cassette d'expression intégrée au génome et est constituée du promoteur inductible du lactose *LAC4,* soit un gène étranger codificateur pour une protéine immunogène, un terminator de transcription et un gène *LAC4* codificateur pour l'enzyme β-galactosidase sous le contrôle du promoteur *GAL80* de *K*. *lactis* (*KIGAL80-P*). L'intégration ciblée s'effectue par les gènes étrangers sur le locus *LAC4* du génome de levure sans que des séquences ADN complémentaires soient introduites ou que l'expression de gènes étrangers soient dosément induits par le lactose ou le galactose ou constitutivement activés après désactivation du gène régulateur *KIGAL80.*

19. Utilisation de la souche recombinante de levure *Kluyveromyces lactis* selon la revendication 18 relative à l'expression de la protéine étrangère.

20. Souche recombinante de levure *Kluyveromyces lactis* selon la revendication 18 relative à l'application en terme de vaccination.

21. Souche recombinante de levure *Kluyveromyces lactis* selon la revendication 18 relative à l'application en terme de vaccination orale/muqueuse pour faire face au virus BVD ou CFS.
